# EUROPÄISCHE PATENTANMELDUNG

(11) **EP 0 778 349 A2**
(43) Veröffentlichungstag der Anmeldung: **11.06.1997**
(21) Anmeldenummer: 96117517.1
(22) Anmeldetag: 31.10.1996
(51) Int. Cl.: C12N 15/85, C12N 5/10, A61K 48/00

(54) **Genkonstrukt und dessen Verwendung**

(30) Priorität: 07.11.1995 DE 19541450
(71) Anmelder: GSF-Forschungszentrum für Umwelt und Gesundheit GmbH, 85764 Oberschleissheim (DE)
(72) Erfinder: Polack, Axel, 80798 München (DE); Hörtnagel, Konstanze, 80634 München (DE); Wolf, Jürgen, 50733 Köln (DE); Mücke, Susanne, 50996 Köln (DE)
(74) Vertreter: Behnisch, Werner, Dr.

(57) **Zusammenfassung**

Die Erfindung offenbart ein Genkonstrukt, mindestens enthaltend, in funktioneller Verbindung,
(a)
   (i) eine Kombination von zwei Enhancerelementen des Immunglobulin-κ-Locus, nämlich den κ-Intron-Enhancer (κ-Ei) und den κ-3'-Enhancer (κ-E3'), oder
   (ii) eine Kombination von zwei Enhancerelementen des schwere Ketten-Immunglobulin-µ-Locus, nämlich µ-Ei und die 3' von Cα gelegene µ-E3'-Enhancerregion, oder
   (iii) eine Kombination eines oder mehrerer dieser Enhancerelemente aus (ii) mit einem oder mehreren der vorgenannten Elemente des Immunglobulin-κ-Locus oder
   (iv) das eine Enhancerelement aus dem Immunglobulin-λ-Locus oder
   (v) eine Kombination dieses Enhancerelements aus (iv) mit einem oder mehreren der vorgenannten Elemente des Immunglobulin-κ-Locus oder
   (vi) eine Kombination dieses Enhancerelements aus (iv) mit einem oder mehreren der vorhergehenden Elemente des schwere Ketten-Immunglobulin-µ-Locus, weiterhin
(b) einen Promotor,
(c) ein Gen von Interesse, ausgewählt aus mindestens einem Element der Gruppe, bestehend aus Zytokin-Gen, virales Antigen, Zelladhäsions-Gen, Tumorantigen, co-stimulatorisches Signalmolekül-Gen, ein nicht mit dem Empfänger identisches HLA-Gen und β-Galactosidase-Gen, und
(d) eine Polyadenylierungsstelle (PAA).

## Beschreibung

Die Erfindung betrifft ein Genkonstrukt, eine pharmazeutische Zubereitung sowie deren Verwendungen.

Die Ära der Gentherapie in der Medizin wurde durch den erfolgreichen Gentransfer des Adenosindesaminase-Gens bei einem schwer immundefizienten Kind eingeleitet. Bisher deuten vor allem tierexperimentelle Befunde darauf hin, daß diese Therapieform nicht nur bei der Korrektur genetisch verursachter Leiden, sondern auch bei der Therapie von malignen Neoplasien zum Einsatz kommen kann (Culver and Blaese, 1994).

Die bisher in der Erprobung befindlichen Verfahren zielen entweder auf eine direkte Abtötung bzw. zumindest eine "Normalisierung" der Tumorzelle oder auf die Aktivierung einer gegen den Tumor gerichteten Immunreaktion. Die Abtötung durch den Transfer sogenannter Suizidgene oder die Normalisierung durch den Transfer von Tumorsuppressorgenen setzen eine hohe Effizienz des Gentransfers voraus. Der direkte intratumorale Transfer von Mauszellinien, die Retroviren mit dem herpesviralen Suizidgen Thymidinkinase produzieren, wurde beim Glioblastoma multiforme bereits durchgeführt (Culver and Van Gilder, 1994).

Solange noch kein effizientes System zur Verfügung steht, das den gezielten Gentransfer in alle Tumorzellen in vivo erlaubt, erscheinen Ansätze, die das Immunsystem zum Aufspüren und Abtöten aller Tumorzellen mit einbeziehen, am aussichtsreichsten. Eine Voraussetzung dieser Ansätze ist jedoch, daß das Immunsystem prinzipiell in der Lage ist, aufgrund von tumorspezifischen Antigenen die Tumorzellen zu erkennen (Boon et al., 1994). Als tumorspezifische Antigene kommen beispielsweise virale Genprodukte (z.B. Genprodukte des Humanen Papillomvirus bei Genitaltumoren) oder durch Mutation veränderte Onkogenprodukte (z.B. das ras-Genprodukt oder das tumorspezifische bcr-abl-Fusionsprotein) in Frage. Weitere gute Kandidaten für tumorspezifische Antigene sind die sogenannten Idiotypen, d.h. Immunglobuline oder T-Zellrezeptoren auf der Zelloberfläche von Tumoren, die sich von B- oder T-Zellen ableiten. In letzter Zeit konnte außerdem eine Reihe von tumorassoziierten Antigenen, z.B. beim malignen Melanom, identifiziert werden. Diese Gene werden jedoch nicht ausschließlich vom Tumor, sondern in geringem Maß auch von anderen somatischen Zellen, wie z.B. den Melanozyten, exprimiert. Die Kenntnis von tumorspezifischen oder tumorassoziierten Antigenen wird voraussichtlich sprunghaft zunehmen, da es kürzlich gelungen ist, mit biochemischen Methoden die im MHC-Komplex eines Tumors präsentierten Peptide zu gewinnen und zu analysieren (Mandelboim et al.; Cox et al., 1994).

Im tierexperimentellen Ansatz wurde bereits Mitte der achtziger Jahre beobachtet, daß Tumorzellen, wenn sie durch Gentransfer bestimmte Zytokine (z.B. IL-2) produzieren, ihre Tumorigenität im syngenen Tier verlieren (Pardoll, 1993). Dieser Effekt war auch zu beobachten, wenn modifizierte und unmodifizierte Zellen gemischt wurden. Die lokale Produktion eines immunstimulatorischen Zytokins eines Teils der Tumorzellen ist offensichtlich in der Lage, eine gegen den Wildtyp-Tumor gerichtete Immunreaktion hervorzurufen. Eine der umfangreichsten Studien dieser Art an dem malignen Melanom-Modell B 16 der Maus zeigte, daß Retroviren, die GM-CSF, IL-4 und IL-6 transduzierten, am wirkungsvollsten waren (Dranoff et al., 1993). Diese Beobachtungen hatten zur Folge, daß weltweit eine ganze Reihe von klinischen Studien begonnen wurden oder derzeit begonnen werden, die einen intratumoralen Transfer von Zytokingenen zum Inhalt haben (Foa et al., 1994). Die Protokolle sehen in der Regel einen ex vivo-Gentransfer in kurzzeitig in vitro etablierten Tumorzellen vor. Als Vektoren kommen in den meisten Protokollen amphotrope Retroviren zum Einsatz. Die Zellen werden nach dem viralen Gentransfer entweder bestrahlt oder unbestrahlt in die Patienten reimplantiert. Diese Ansätze werden jedoch stark limitiert durch die technischen Schwierigkeiten, Tumorzellen in vitro auch nur kurzzeitig zu kultivieren. Eine Modifikation dieses Ansatzes sieht daher vor, nicht die Tumorzellen selbst, sondern entweder tumorinfiltrierende Lymphozyten (TIL) (Treisman et al., 1995) oder autologe Fibroblastenzellen zu transduzieren bzw. zu transfizieren.

Ein weiterer Ansatz, der ebenfalls auf Beobachtungen im Tierexperiment in den 80er Jahren zurückgeht, wurde von Gary Nabel erarbeitet und bereits in ein klinisches Protokoll umgesetzt (Nabel et al., 1993; Plautz et al., 1993). Dieser Ansatz geht davon aus, daß eine artifizielle Allogenisierung eines Teils der Tumorzellen durch den Transfer von Transplantationsantigenen ausreichend sein könnte, eine Immunantwort des Organismus auf die unmodifizierte Tumorzelle hervorzurufen. Dieses Protokoll sieht den Transfer eines HLA-B7-Expressionskonstrukts mittels Liposomen direkt in den Tumor vor. Die mehrmalige Injektion des HLA-B7-Genkonstrukts in Hautmetastasen eines moribunden Patienten erzeugte eine Regression einer unbehandelten anderen Hautmetastase sowie einer Lungenmetastase (Nabel et al., 1993).

Auf vielen Tumorzellen in vivo fehlt das Oberflächenantigen B7, das costimulatorische Signale für die Erkennung durch T-Zellen vermittelt. Es wird daher versucht, Tumorzellen durch Gentransfer zur Produktion dieses Signalmoleküls anzuregen (June et al., 1994; Li et al., 1994).

Die oben beschriebenen Lösungsansätze weisen u.a. die folgenden Nachteile auf:

### RETROVIRALE VEKTOREN:

Amphotrope Retroviren haben den Vorteil, daß die Integration der proviralen DNA in die Zielzelle und die virale Promotor/Enhancer-Kombination in der Regel ein über mehrere Zellteilungen stabiles Expressionsniveau erlaubt. Die notwendige Integration birgt jedoch die Gefahr der Insertionsmutagenese in sich. Darüber hinaus produzieren sogenannte "packaging"-Zellinien relativ geringe Mengen an rekombinantem Virus, die sich bisher aufgrund ihrer Labilität nicht anreichern lassen. Für den direkten intratumoralen Gentransfer kommen daher nur virusproduzierende Zellen in Frage. Die Freisetzung von infektiösem Virus im Zielorganismus kann jedoch nach hämatogener Verschleppung auch zur Infektion anderer sich teilender Zellen wie z.B. Darmepithel oder hämatopoetischer Stammzellen führen.

### DIREKTER INTRATUMORALER DNA-TRANSFER:

Die durch Liposomen vermittelte direkte Einschleusung von DNA wurde erfolgreich am Endothel von großen Gefäßen gezeigt (Ohno et al., 1994). Dieser Ansatz hat zwar den Vorteil, daß er weder das Risiko der Insertionsmutagenese noch der unerwünschten Fernwirkung beinhaltet; dieser Ansatz führt in sich teilendem Gewebe jedoch nur zu einer transienten Expression des eingeschleusten Genkonstrukts, da die DNA in der Regel weder integriert noch repliziert wird.

### TUMORZELLKULTUR UND EX VIVO-TRANSFEKTION:

Die Kultivierung der Tumorzellen in vitro von jedem einzelnen Patienten stellt eines der größten technischen Probleme dar. Der Gentransfer in relativ kurzzeitig in Kultur befindliche Tumorzellen erfordert höchstes experimentelles Geschick und gelingt nur in einem Teil der Fälle. Die Infektion mit rekombinanten Retroviren stellt bisher die Technik der Wahl für einen Gentransfer in diese Art Zellen dar.

### EBV, EBV-abgeleitete Vektoren und EBV-immortalisierte Zellen

Das EBV liegt in den LCLs im Zustand der Latenz vor. Dies bedeutet, daß nur ein sehr geringer Prozentsatz der infizierten Zellen infektiöses Virus produziert. Im Zustand der Latenz werden lediglich sechs nukleär lokalisierte (EBNA1,2,3A,B,C, -LP) und zwei membranständige Proteine (LMP und TP) des Virus exprimiert. Das Genom des EBV liegt in der infizierten Zelle in der Regel episomal mit einer Kopienzahl zwischen 10 und 100 vor. Im Zustand der Latenz geht die Replikation des viralen Genoms von einem Replikationsursprung (oriP) aus (Yates et al., 1984). Zur Aufrechterhaltung der episomalen Replikation ist weiterhin die Bindung des EBNA1-Proteins an den oriP notwendig (Yates et al., 1985). Die von EBV abgeleiteten Vektoren bestehen aus pBR-Sequenzen, oriP, einer Expressionskassette für EBNA1 und einem Selektionsmarker für eukaryonte Zellen (z.B. das Hygromyzinresistenzgen). Diese Vektoren besitzen darüber hinaus noch die Kapazität für 20 bis 30 kb weitere Fremd-Sequenzen. Konstrukte, die auf diesen Vektoren basieren, werden in der Zelle: (i) auch ohne Selektion sehr viel besser "zurückgehalten" (Middleton und Sugden, 1994), (ii) erlauben eine geregelte Expression frei von Positionseffekten und (iii) besitzen ein wesentlich vermindertes Risiko der Insertionsmutagenese. Die Effizienz, mit diesen Vektoren stabil transfizierte Zellen zu erhalten, ist im Vergleich zu reinen Plasmidvektoren wesentlich höher.

Über 95% der Erwachsenen sind mit EBV infiziert. Die Primärinfektion erfolgt entweder inapparent oder in Form einer infektiösen Mononukleose. Die immunologische Kontrolle der virusinfizierten Zellen in vivo ist sehr gut untersucht. Die verschiedenen Latenzgenprodukte des Virus werden durch spezifische zytotoxische T-Zellen erkannt. Lediglich im Zustand extremer Immunsuppression, die z.B. beim AIDS-Patienten beobachtet oder bei Transplantatempfängern iatrogen erzeugt wird, kann es zur polyklonalen Proliferation EBV-positiver Zellen kommen.

### Genexpressionsverstärkung mitttels Immunglobulin-Regelelementen

Die synergistische Funktionsweise der drei regulatorischen Elemente (κMAR, κEi, κE3') des Immunglobulin-κ-Locus wurde zuerst für die Aktivierung der c-myc-Promotoren P1 und P2 gezeigt (Polack et al., 1993; Hörtnagel et al., 1995). Bei einer beim Burkitt-Lymphom (BL) beobachteten Chromosomentranslokation kommt es zu einer Kolokalisation des c-myc-Gens und der 3' von der "κ joining region" gelegenen Region des humanen Immunglobulin-K-Locus. Es kommt dadurch zu einer Aktivierung des c-myc-Gens. Diese Aktivierung ist gekennzeichnet durch eine Änderung in der Promotorenbenutzung des c-myc-Gens: der P1-Promotor wird stärker benutzt als der P2-Promotor. In normalen, nicht transformierten Zellen wird dagegen bevorzugt der P2-Promotor benutzt. Weiterhin wird eine Aufhebung der Kontrolle des c-myc-Gens auf der Ebene der Transkriptelongation beobachtet.

Um den Mechanismus der c-myc-Aktivierung durch t(2;8)-Translokation zu studieren, wurde die Interaktion c-myc-Gen mit verschiedenen Bereichen des Igκ-Locus untersucht. Als Methode wurde die stabile Transfektion von BL-Zellen mit Hilfe der episomal replizierenden, von EBV abgeleiteten Vektoren gewählt. In diese Vektoren wurde das c-myc-Gen unter der Kontrolle von zwei Bereichen des Igκ-Locus einkloniert. Der eine Bereich erstreckt sich von der J-Region bis ca. 1,2 kb 3' von der konstanten Region (Cκ), während der zweite den 12 kb 3' von Cκ gelegenen κ3'E umfaßt. Die Messung der c-myc-Expression von diesem Konstrukt und verschiedenen Verkürzungen (Deletionen) ergaben zunächst, daß diese Bereiche notwendig und hinreichend für die BL-spezifische Aktivierung des c-myc-Gens ist (Polack et al., 1993). Durch weitere Deletionen konnte gezeigt werden, daß drei Elemente aus dem Igκ-Locus für die Aktivierung des c-myc-Gens verantwortlich sind: κMAR, κEi und κE3' (Hörtnagel et al., 1995). Weiterhin wurde die Chromatinstruktur der stabil in die BL-Zellen eingeführten Konstrukte mit Hilfe der Kartierung DNaseI hypersensitiver Stellen (HSS) untersucht. Sowohl in der 5'-Region des c-myc-Gens als auch in den Anteilen des Igκ-Locus bildeten sich die typischen HSS aus. Dies zeigt, daß sich auf den extrachromosomal replizierenden Konstrukten eine normale Chromatinstruktur ausbildet.

Die Verwendung der Igκ-Elemente im Zusammenhang mit einem episomalen Vektor zeigt:
- eine synergistische Aktivierung eines heterologen Promotors (c-myc) durch die Igκ-Elemente;
- eine große Kapazität der episomalen Vektoren;
- die Ausbildung einer geordneten Chromatinstruktur.

Die in der Veröffentlichung von Hörtnagel et al., 1995, beschriebenen Konstrukte waren Deletionskonstrukte, um die zur c-myc-Aktivierung essentiell notwendigen Ig-Bereiche zu ermitteln. Die erfindungsgemäßen Genkonstrukte mit ihren in funktioneller Anordnung enthaltenen Elementen und die Anwendungen der erfindungsgemäßen Genkonstrukte werden hierdurch nicht nahegelegt.

Es ist eine Aufgabe der vorliegenden Erfindung, ein Genkonstrukt für die Gentherapie bereitzustellen, das die oben genannten, aus dem Stand der Technik bekannten Nachteile vermeidet.

Diese Aufgabe wird erfindungsgemäß durch ein Genkonstrukt gelöst, das, in funktioneller Verbindung, mindestens enthält:
(a)
   (i) eine Kombination von zwei Enhancerelementen des Immunglobulin-κ-Locus, nämlich den κ-Intron-Enhancer (κ-Ei) und den κ-3'-Enhancer (κ-E3'), oder
   (ii) eine Kombination von zwei Enhancerelementen des schwere Ketten-Immunglobulin-µ-Locus, nämlich µ-Ei und die 3' von Cα gelegene µ-E3'-Enhancerregion, oder
   (iii) eine Kombination eines oder mehrerer dieser Enhancerelemente aus (ii) mit einem oder mehreren der vorgenannten Elemente des Immunglobulin-κ-Locus oder
   (iv) das eine Enhancerelement aus dem Immunglobulin-λ-Locus oder
   (v) eine Kombination dieses Enhancerelements aus (iv) mit einem oder mehreren der vorgenannten Elemente des Immunglobulin-κ-Locus oder
   (vi) eine Kombination dieses Enhancerelements aus (iv) mit einem oder mehreren der vorhergehenden Elemente des schwere Ketten-Immunglobulin-µ-Locus, weiterhin
(b) einen Promotor,
(c) ein Gen von Interesse, ausgewählt aus mindestens einem Element der Gruppe, bestehend aus Zytokin-Gen, virales Antigen, Zelladhäsions-Gen, Tumorantigen, co-stimulatorisches Signalmolekül-Gen, ein nicht mit dem Empfänger identisches HLA-Gen und β-Galactosidase-Gen, und
(d) eine Polyadenylierungsstelle (PAA).

Bevorzugt enthält das erfindungsgemäße Genkonstrukt die nachfolgenden Kombinationen von Regulationselementen:
κ-Ei mit λ-E, µ-Ei mit κ-E3', µ-Ei mit µ-E3', κ-Ei mit µ-E3', µ-Ei mit λ-E, κ-MAR mit κ-Ei mit κ-E3', λ-E mit κ-E3', λ-E mit µ-E3'.

Das erfindungsgemäße Genkonstrukt enthält in einer weiteren, bevorzugten Ausführungsform zusätzlich zu einem oder mehreren der obengenannten Enhancerelemente oder der Kombinationen der Enhancerelemente die Immunglobulin-κ-Matrix-Attachment-Region (κ-MAR) oder die Immunglobulin-µ-Matrix-Attachment-Region (µ-MAR).

Das erfindungsgemäße Genkonstrukt ist zur Gentherapie bei Erkrankungen des B-Zell-Systems einsetzbar.

Das Gen von Interesse kann bevorzugt das B7-1- oder B7-2-Gen sein.

Das erfindungsgemäße Genkonstrukt enthält bevorzugt von EBV-Vektoren, mini-EBV-Vektoren, bakteriellen Vektoren, von Retroviren, von Adenoviren assoziierten Viren, von Adenoviren und von Vaccinia-Viren und abgeleitete Sequenzen. Weiterhin bevorzugt enthält es Sequenzen, die sich von bakteriellen Vektoren ableiten. Die von EBV-Vektoren oder mini-EBV-Vektoren abgeleitete Sequenz ist in einer Ausführungsform der Erfindung der Replikationsursprung (oriP).

In einer bevorzugten Ausführungsform enthält das Genkonstrukt der vorliegenden Erfindung zusätzlich die Expressionskassette für EBNA1. Weiterhin zusätzlich kann das erfindungsgemäße Genkonstrukt ein Markergen enthalten, das bevorzugt ein Resistenzgen ist. Dieses Resistenzgen wird aus an sich bekannten Resistenzgenen ausgewählt. Beispielsweise kann ein Ampicillinresistenzgen oder ein Hygromyzinresistenzgen oder ein Neomycinresistenzgen eingesetzt werden.

Die bakterielle Vektorsequenz kann aus beliebigen, an sich bekannten Vektorsequenzen ausgewählt werden. Bevorzugt ist sie von pBR-Vektoren abgeleitet.

Das erfindungsgemäße Genkonstrukt umfaßt in einer besonders bevorzugten Ausführungsform von EBV abgeleitete Vektorsequenzen, mit oder ohne dem EBNA1-Gen. Auf die Veröffentlichung von Sugden et al, 1985, wird hier vollinhaltlich verwiesen, und sie wird zur vollständigen Offenbarung in diese Anmeldung mitaufgenommen.

Der Promotor kann aus beliebigen, in der vorliegenden, erfindungsgemäßen funktionellen Verbindung verwendbaren Promotoren ausgewählt werden. Bevorzugt können Promotoren aus der Gruppe B-Zell-spezifischer Promotoren, Zytomegalievirus-(CMV-)-, tk- und β-Globin-Promotoren verwendet werden.

Die Polyadenylierungsstelle kann aus solchen Sequenzen abgeleitet werden, die üblicherweise als Polyadenylierungsstelle eingesetzt werden. Ein Beispiel hierfür ist die Polyadenylierungsstelle des humanen β-Globin-Gens oder des SV40-Polyomavirus.

Das erfindungsgemäße Genkonstrukt ermöglicht die Expression therapeutisch einsetzbarer Gene. Bevorzugt werden diese therapeutisch einsetzbaren Gene in B-Zellen, in von B-Zellen abgeleiteten Zellen, beispielsweise B-Zell-Tumorzellen oder mit EBV oder mini-EBV-immortalisierten Zellen, nach erfolgreichem Gentransfer exprimiert, um maligne und virale Erkrankungen zu therapieren.

Beispiele für die durch das erfindungsgemäße Genkonstrukt exprimierbaren Gene sind die Zytokin-Gene, ausgewählt aus der Gruppe von IL-2, -4, -6, -7, -8, -10, GM-CSF, G-CSF, TNF-alpha, MCP-1, Interferon-gamma.

In einer weiteren Ausführungsform der Erfindung werden in das erfindungsgemäße Konstrukt als Gen von Interesse Tumorantigene aus der Gruppe von Antigenen des Humanen Papillom-Virus (HPV), mit malignem Melanom assoziierten Antigenen, der Familie der MAGE-, BAGE- und GAGE-Gene, dem gp100, den Idiotypen von T-Zell- oder B-Zell-Rezeptoren von Tumoren und mutierten Onkogenen eingesetzt. Die Tumorantigene können insbesondere tumorassoziierte oder tumorspezifische Antigene sein.

Die oben genannten Tumorantigene sind bevorzugt mutierte Onkogene, beispielsweise ras- oder p53-Gene oder deren Derivate. Das mit malignem Melanom assoziierte Antigen ist bevorzugt das Tyrosinase-Gen. Weitere, erfindungsgemäß einsetzbare Gene von Interesse sind dem Fachmann bekannt und in Abhängigkeit von der zu behandelnden Erkrankung auswählbar.

Die Erfindung umfaßt auch Prokaryontenzellen und Eukaryontenzellen, die mit einem der erfindungsgemäßen Genkonstrukte transfiziert wurden, so daß sie das Genkonstrukt in integrierter oder episomaler, d.h. nicht-integrierter Form enthalten. Bevorzugt wird als Prokaryontenzelle eine E. coli-Zelle eingesetzt und als Eukaryontenzelle eine mit EBV oder mini-EBV-immortalisierte B-Zelle.

Das erfindungsgemäße Genkonstrukt kann als Gen von Interesse bevorzugt die viralen Antigene des HIV, des CMV, die eine Immunantwort gegen die Virus infizierte Zelle auslösen können, des HTLV1 und des HPV enthalten.

Die erfindungsgemäßen Genkonstrukte sind in Form einer pharmazeutischen Zubereitung einsetzbar, die weiterhin an sich bekannte und übliche Träger- und/oder Hilfsstoffe enthält.

Die erfindungsgemäßen Genkonstrukte liegen in der erfindungsgemäßen pharmazeutischen Zubereitung bevorzugt in Liposomen oder Liposom-ähnlichen Strukturen vor.

In einer weiteren Ausführungsform der Erfindung enthält das erfindungsgemäße Genkonstrukt kein c-myc-Tumorantigen.

In einer bevorzugten Ausführungsform der Erfindung wird eine pharmazeutische Zubereitung bereitgestellt, die ein erfindungsgemäßes Genkonstrukt in einer wirksamen Menge neben üblichen Träger- und/oder Hilfsstoffen enthält.

In einer weiteren bevorzugten Ausführungsform der Erfindung wird eine pharmazeutische Zubereitung bereitgestellt, die mit einem erfindungsgemäßen Genkonstrukt modifizierte primäre, mit EBV oder mini-EBV-immortalisierte B-Lymphozyten oder Fibroblastenzellen in einer wirksamen Menge neben üblichen Träger- und/oder Hilfsstoffen enthält.

In einer weiteren bevorzugten Ausführungsform wird eine pharmazeutische Zubereitung bereitgestellt, die mit einem erfindungsgemäßen Genkonstrukt modifizierte primäre, mit EBV oder mini-EBV-immortalisierte B-Lymphozyten oder Fibroblastenzellen zusammen mit autologen T-Zellen in einer wirksamen Menge neben üblichen Träger- und/oder Hilfsstoffen enthält.

In einer weiter bevorzugten Ausführungsform wird eine pharmazeutische Zubereitung bereitgestellt, die autologe T-Zellen enthält, die ex vivo unter Verwendung primärer, mit EBV oder mini-EBV-immortalisierter B-Lymphozyten oder Fibroblasten, die mit einem erfindungsgemäßen Genkonstrukt modifiziert wurden, stimuliert und expandiert wurden.

Durch die Verwendung eines von EBV abgeleiteten Vektors in Kombination mit drei spezifischen Regulationselementen des Immunglobulin-kappa-Locus, nämlich der Matrix-Attachment-Region, des Intron-Enhancers und des 3'-Enhancers, kann spezifisch in B-Zellen ein therapeutisch wertvolles Gen über einen längeren Zeitraum exprimiert werden. Ein weiterer Vorteil des Einsatzes der erfindungsgemäßen Genkonstrukte liegt darin, daß die experimentell sehr aufwendige Kultur primärer Zellen entfällt, da durch die Infektion von peripheren B-Zellen mit EBV oder mini-EBV- von fast jedem Individuum immortalisierte Zellen (LCL-Zellen) gewonnen werden können (Übersicht in: Rogers et al., 1992). Die Etablierung derartiger LCLs ist experimentell wesentlich weniger aufwendig als die Kultur primärer Tumorzellen.

Die Erfindung wird nachfolgend anhand der beiliegenden Figuren näher erläutert. Die Figuren zeigen:
- Fig. 1: die Konstruktion und die Struktur eines erfindungsgemäßen Genkonstrukts.
- Fig. 2: die schematische Darstellung der Klonierung eines zu exprimierenden Gens in den Vektor BC230 oder einem davon abgeleiteten Vektor.
- Fig. 3: die β-Galactosidase-Expression in BL-Zellen, die mit DZ17 bzw. CMV-βGal transfiziert wurden.

Die erfindungsgemäßen Genkonstrukte basieren bevorzugt auf Vektoren, die sich vom Epstein-Barr-Virus ableiten und die Sequenzen für oriP, die Expressionskassette für EBNA1, Resistenzmarker und wahlweise weiterhin pBR-Sequenzen enthalten.

Die erfindungsgemäßen Vektoren enthalten weiterhin bevorzugt eine Kombination verschiedener regulatorischer Elemente aus dem Immunglobulin-kappa-Locus: die Matrix-Attachment-Region (MAR) und die Enhancer-Elemente, die im Intron (Ei) des kappa-Locus und 12 kb 3' (E3') vom konstanten Teil des Ig-kappa-Gens lokalisiert sind.

Der Promotorbereich der Expressionskassette stammt bevorzugt vom humanen Zytomegalievirus (immediate early gene; CMV-P). Die Polyadenylierungsstelle stammt bevorzugt vom humanen β-Globin-Gen. Als Promotoren sind weiterhin Minimalpromotoren, beispielsweise der tk- oder β-Globin-Promotor, einsetzbar.

In einer bevorzugten Ausführungsform soll die Aktivität des Vektors auf B-Zellen begrenzt werden, um sicherheitstechnische Erfordernisse zu erfüllen. Dies kann beispielsweise durch die Einführung des c-myc P1-Promotors anstelle des CMV-Promotors erreicht werden. Es konnte gezeigt werden, daß dieser Promotor ohne Enhancer fast keine Aktivität besitzt. Diese Eigenschaft läßt diesen Promotor besonders geeignet erscheinen, mit Hilfe B-Zell-spezifischer Enhancer eine Begrenzung der Aktivität des Vektors auf die B-Zellen zu erreichen.

Die Sequenz der Expressionskassette für EBNA1 ist an sich bekannt. Diese Expressionskassette kann beispielsweise in einen von EBV abgeleiteten Vektor, wie er beispielsweise von Yates et al. 1985 beschrieben wurde, einkloniert werden. Besonders geeignet ist die Region zwischen dem oriP und der Hygromyzin-Resistenzgen-Kassette (HYG).

Abgewandelte Vektoren, die als Reportergen entweder das β-Galaktosidase- oder Chloramphenicol-Acetyltransferase-Gen unter der Kontrolle des CMV-Promotors tragen, sind möglich. Für die Transfektion von EBV-negativen Zellen sind solche Vektoren geeignet, die eine Expressionskassette für das EBNA1-Gen tragen.

In die Klonierungsstellen SfiI 842 bis SfiI 862 oder NaeI bei 850 können verschiedene Zytokin-Gene oder Tumorantigene einkloniert werden. Erfindungsgemäß verwendbare Zytokin-Gene sind beispielsweise: IL-2, -4, -6, -7, -8, -10, GM-CSF, G-CSF, TNF-alpha, MCP-1, Interferon-gamma.

Als virale Antigene oder Tumorantigene kommen in Frage: virale Antigene von Viren, die mit der Entstehung von Tumoren assoziiert sind, wie z.B. Antigene des Humanen Papillom-Virus (HPV), mit malignem Melanom assoziierte Antigene wie z.B. Tyrosinase, die Familie der MAGE-, BAGE- und GAGE-Gene und das gp100, die Idiotypen von T-Zell- oder B-Zell-Rezeptoren von Tumoren, die sich von T- oder B-Zellen ableiten, mutierte Onkogene wie z.B. das ras- oder p53-Gen etc.. Als virale Antigene kommen weiterhin in Frage HIV-Antigene und CMV-Antigene, die eine Immunantwort gegen die virusinfizierte Zelle auslösen können.

Für den direkten oder ex vivo-Gentransfer in Tumorzellen kann auch ein für den Empfänger fremdes HLA-Gen, z.B.- HLA-B7-Gen, oder aus das Gen des co-stimulatorisch wirksamen Moleküls B7 zum Einsatz kommen.

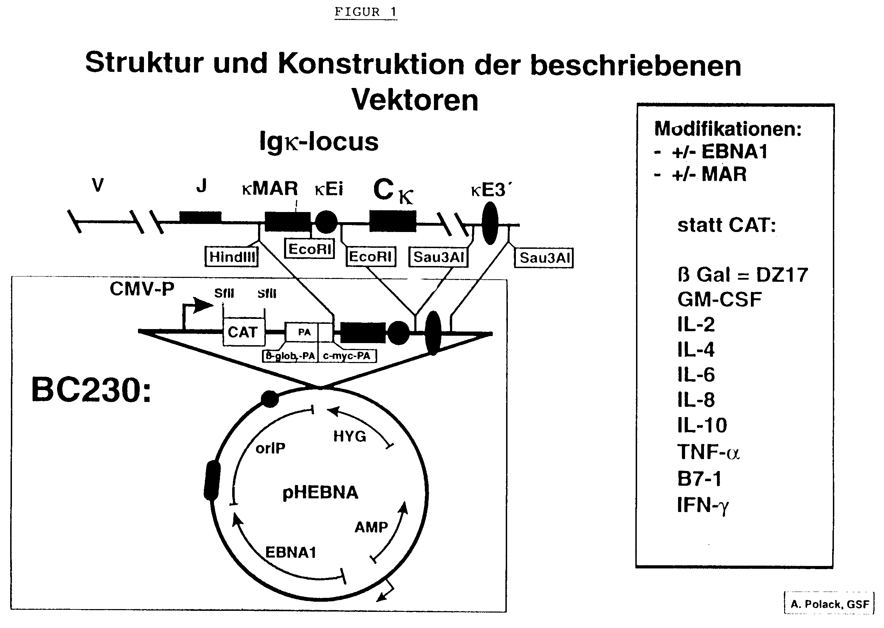

Bezugnehmend auf die Fig. 1 wird nachfolgend beispielhaft die Konstruktion eines erfindungsgemäßen Genkonstrukts beschrieben, nämlich des Vektors BC230A.

Ausgangspunkt der Konstruktion von BC230 war der von Sugden et al. (1985) beschriebene vom Epstein-Barr-Virus abgeleitete Vektor p201 (pHEBNA). Dieser Vektor enthält neben Teilen von pBR322 und einer Expressionskassette für das Hygromyzin-Resistenzgen (hph) den plasmidalen Replikationsursprung (oriP) von EBV (Sequenzposition 7333 bis 9109 von B95-8; EBV [= Bezeichnung in der EMBL-Datenbank] und eine 2,6 kbp-Region (Sequenzposition 107567 bis 110176 von B95-8), die für das EBNA1-(Epstein-Barr-Virus nuclear antigene 1-) Gen kodiert.

Zwischen oriP und dem Hygromyzin-Resistenzgen wurde dann der Promotor/Enhancer-Bereich des humanen Zytomegalievirus (Sequenzposition von 467 bis 1218 von HEHCMVP1 [= Bezeichnung in der EMBL-Datenbank]), das Chloramphenicol-acetyl-Transferase-Gen (CAT) (Sequenzposition 452 bis 1240 von BLCAT3DNA [= Bezeichnung in der EMBL-Datenbank]) und die Polyadenylierungsstelle des Kaninchen beta-Globin-Gens (Sequenzposition 31393 bis 32554 von OCBGLO01 [= Bezeichnung in der EMBL-Datenbank]) einkloniert.

Zwischen das β-Globin-Polyadenylierungssignal und das Hygromyzin-Resistenzgen wurde ein Teil der Polyadenylierungsstelle von c-myc (Sequenzposition 7800 bis 8056 von HSMYCKOB [= Bezeichnung in der EMBL-Datenbank]), die Matrix-Attachment-Region (κMAR), der angrenzende Intron-Enhancer (κEi) und der 3' von der konstanten Region des humanen kappa-Gens lokalisierte 3'-Enhancer (κ3'E) eingefügt. DAS κMAR-Element wurde als HindIII-EcoRI-Fragment (Sequenzposition 3237 bis 3447 HSIGK1 [= Bezeichnung in der EMBL-Datenbank]) und der κEi als EcoRI-EcoRI-Fragment (Sequenzposition 3447 bis 4153 HSIGK1 [= Bezeichnung in der EMBL-Datenbank]) definiert (Hörtnagel et al., 1995). Der κ3'E entspricht einem Sau3AI-Sau3AI-Fragment von 1169 bp Länge.

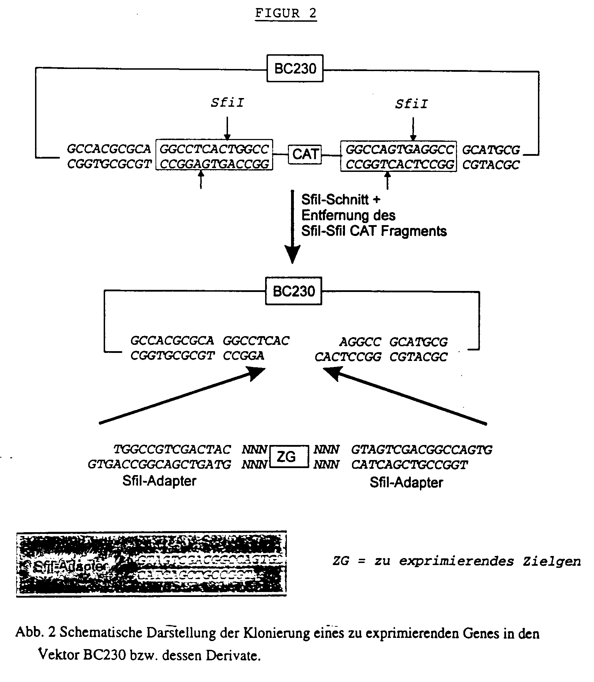

Das CAT-Gen kann durch Schnitt mit dem Restriktionsenzym SfiI entfernt werden. Die dadurch entstehenden Enden des Vektorfragmentes (BC230vec) sind nicht kompatibel und können daher nicht mit sich selbst ligiert werden. Die Behandlung des Vektorfragmentes mit Phosphatase (CIP; calf intestine phosphatase) entfällt daher. Ein zu exprimierendes Zielgen, das als "blunt end"-Fragment vorliegt (entweder durch geeigneten Restriktionsenzymverdau (Enzyme, die stumpfe Enden ["blunt ends"] erzeugen) oder durch Restriktionsenzymverdau, gefolgt von einer Behandlung mit Klenow-Polymerase, um überstehende Enden aufzufüllen), kann durch Ligation mit zwei SfiI-Adaptern (siehe Abb. 2) in BC230vec einkloniert werden. Durch die Inkompatibilität der auf der einen Seite des Adapters erzeugten überstehenden Enden entstehen bei der Ligation keine unerwünschten zirkelförmigen Moleküle. Ein weiterer Vorteil dieses Vorgehens besteht darin, daß im zu klonierenden Genabschnitt die Erkennungssequenz für das Enzym SfiI vorkommen kann. Der zu klonierende Genabschnitt muß nicht vor der Ligation in den Vektor mit SfiI geschnitten werden, wie es zur Vorbereitung einer "klassischen" Linker-Ligation notwendig ist.
Durch Modifikationen des Vektors BC230 entstanden folgende Varianten:
- ohne MAR Element: das HindIII-EcoRI-Fragment wurde entfernt (BC229);
- ohne EBNA1-Gen für die Expression in EBV-positiven Zellen (BC243);
- mit dem β-Galaktosidase-Gen anstelle des CAT-Gens (DZ17);
- folgende Gene können beispielsweise in den Vektor BC230 einkloniert werden: IL-2, -4, -6, -8, -10, GM-CSF, B7-1, IFN-γ, TNF-α; diese Vektoren werden dann als BC219 bezeichnet.

### Ausführungsbeispiel 1:

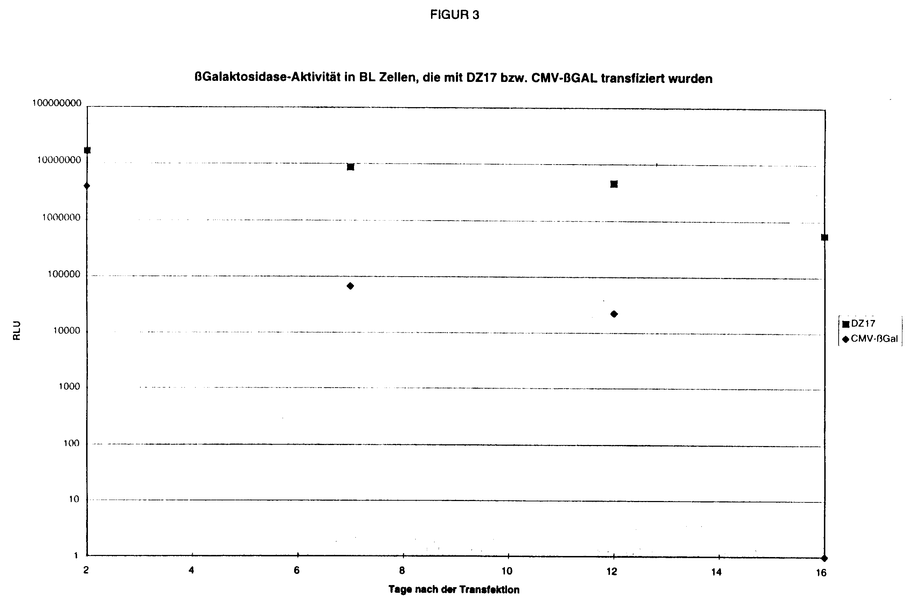

Die Fig. 3 zeigt die Vorteile der Anwendung des erfindungsgemäßen Vektors am Beispiel von DZ17, der das β-Galaktosidase-Gen enthält.

BL-Zellen (Raji) wurden durch Elektroporation mit dem Vektor DZ17 und dem CMV-β-Gal-Konstrukt (ohne Enhancer-Kassette und ohne EBV-Anteile; β-Gal = β- Galactosidase) transient transfiziert. Die Zellen wurden anschließend für 16 Tage ohne Zusatz von Hygromyzin (d.h. ohne Selektion) kultiviert. Nach 2, 7, 12 und 16 Tagen wurden Aliquots der Zellen gewonnen und die β-Galaktosidase-Expression bestimmt. Fig. 3 zeigt das Ergebnis dieses Versuchs. Während nach 2 Tagen sich die Expression der beiden Plasmide um ca. den Faktor 4 unterschied, zeigte sich ein deutlicher Unterschied bereits nach 7 Tagen. Nach 16 Tagen war die Expression von β-Galaktosidase in den Zellen, die mit CMV-β-Gal transfiziert waren, nicht mehr nachweisbar. In Zellen, die mit DZ17 transfiziert waren, konnte weiterhin eine zwar um ca. 10-fach geringere, aber dennoch hohe β-Galaktosidase-Aktivität gemessen werden. Dieses Ergebnis muß dahingehend interpretiert werden, daß der erfindungsgemäße Vektor die Eigenschaft erfüllt, in Zellen zurückgehalten zu werden, eine hohe Expression zu ermöglichen und eine längere Expression zu gewährleisten.

### Ausführungsbeispiel 2:

### TRANSFEKTIONSEFFIZIENZ:

Eine EBV-positive (BL60) und eine EBV-negative (BJAB) humane Lymphomzellinie wurden als Zielzelle für den Vergleich eines herkömmlichen integrierenden Vektors (pKEX, Rittner et al., 1991) mit dem Vektor BC219, einem Derivat von BC230, das kein CAT-Gen enthält, gewählt. BC219 wurde anstatt BC230 eingesetzt, weil BC230 Chloramphenicol-Acetyl-Transferase (CAT) exprimiert. Dieses Enzym könnte in hohen Dosen in der Zelle toxische Wirkung besitzen und somit den Vergleich erschweren. pKEX enthält den CMV-Promotor/Enhancer Bereich, eine von SV40 abgeleitete Polyadenylierungsstelle, die Expressionskassette für das Hygromyzin-Resistenzgen und ein pBR-Derivat zur Vermehrung in Bakterien. Dieser Vektor und seine Derivate müssen in das zelluläre Genom integrieren, um stabil in den Zielzellen repliziert zu werden.

DNS von BC219 und pKEX wurden durch Elektroporation in die beiden Zellinien eingeschleust (Methode beschrieben in Polack et al., 1991). Nach 24h Stunden ohne Selektionsdruck, wurden die Zellen bei einer Dichte von 2 x 10⁵ Zellen/ml in eine 96 Loch-Zellkulturplatte bei einem Volumen von 100 µl/Loch unter Selektionsbedingungen (400 µg/ml Hygromyzin) ausgesät. Die Ansätze, in denen sich Zellproliferation zeigte, wurden gezählt, vermehrt und weiter untersucht. Die folgende Tabelle zeigt das Ergebnis eines solchen Experimentes:

| TABELLE 1 | | |
|---|---|---|
| Zellinie | Vektor | Transfektionseffizienz: pro 1x10⁶ ausplattierter Zellen wuchsen aus: |
| BJAB | pKEX | 10 |
| | BC219 | 100 |
| BL60 | pKEX | 0,3 |
| | BC219 | 500 |

Dieses Ergebnis läßt sich dahingehend interpretieren, daß mit dem BC230-Derivat BC219 sehr viel einfacher stabile Transfektanten hergestellt werden können.

### Ausführungsbeispiel 3:

### ZYTOKINEXPRESSION MIT HILFE VON BC230-DERIVATEN:

Wie in der Fig. 1 gezeigt, wurden die cDNAs von TNFa, GM-CSF und IL6 in den Vektor BC230 bzw. das Derivat BC219 sowie pKEX einkloniert.

Mit Hilfe dieser Konstrukte wurden nach stabiler Transfektion und Selektion mit Hygromyzin verschiedene Transfektanten erzeugt. Die Produktion der verschiedenen Zytokine wurde mit Hilfe eines kommerziell erhältlichen ELISA (Fa. Biermann, Bad Nauheim, Deutschland) im Zellüberstand der verschiedenen Transfektanten bestimmt. Die BL60-Zellen produzieren weder spontan noch nach Transfer von BC230 oder pKEX eines dieser Zytokine. Mit den integrierenden Vektoren (pKEX und Derivate) hergestellte Transfektanten produzierten in keinem Fall eine messbare Menge an TNFα, GM-CSF oder IL6.

Die zehn verschiedenen Sublinien, die mit jedem der drei Zytokinvektoren auf Basis des BC219-Vektors erzeugt wurden, sezernierten alle ungefähr gleiche Mengen an Zytokin in den Überstand. Die Schwankungsbreite der Zytokinproduktion der einzelnen Transfektanten ist in der folgenden Tabelle angegeben:

**TABELLE 2**

| Zellinie | Konstrukt | Anzahl der analysierten Klone | Zytokinproduktion (Schwankungsbreite) |
|---|---|---|---|
| BL60 | pKEX-CAT | 10 | nicht detektierbar |
| | pKEX-IL6 | 5 | " |
| | pKEX-TNFα | 9 | " |
| | BC230 | 10 | " |
| | BC219-TNFα | 10 | 1500-2000 pg/ml TNFa |
| | BC219-GM-CSF | 10 | 700 pg/ml GM-CSF |
| | BC219-IL6 | 10 | 900-1000 pg/ml IL6 |

EBV-abgeleitete Vektoren besitzen die Eigenschaft, nach Transfer in Zellen entweder ex vivo oder in vivo ohne Selektion zurückgehalten zu werden. Dadurch kommt es über einen längeren Zeitraum zu einer Expression von Genen, die mit Hilfe dieser Vektoren übertragen wurden. Der direkte intratumorale Transfer eines beispielsweise GM-CSF-exprimierenden Konstrukts, z.B. in ein B-Zell-Lymphom zur Stimulation einer körpereigenen Abwehr gegen die Tumorzellen mit Hilfe von Liposomen, ist daher ein wirkungsvolles Therapeutikum.

### EBV-immortalisierte B-Zellen als Zielzellen für den Gentransfer

Die Kultivierung von Tumorzellen in vitro ist nur bei einem Teil der Tumorpatienten erfolgreich. Mit Hilfe des EBV lassen sich von jedem Individuum in unbegrenzter Menge immortalisierte B-Zellen herstellen. Diese Zellen können durch Gentransfer mit dem oben genannten Konstrukt zur Produktion immunmodulatorisch wirksamer Botensubstanzen (Zytokine) gebracht werden. Diese so erzeugten Zellen können zur Stimulation einer Immunreaktion gegen einen Tumor direkt in den Tumor oder zusammen mit Tumorzellen, z.B. Subkutan, inokuliert werden. Zur Sicherheit können diese Zellen vor Transplantation mit Hilfe einer Bestrahlung an der weiteren Proliferation gehindert werden.

Weiterhin können EBV-immortalisierte Zellen durch Transfektion von entsprechenden Genkonstrukten zur Expression von viralen oder Tumor-antigenen gebracht werden. Diese Antigene sollten von den Zellen in MHC-restringierter Weise präsentiert werden. Daher eignen sich diese Zellen zur Stimulation von autologen T-Zellen, die diese Antigene erkennen. Die so stimulierten T-Zellen können mit Hilfe dieser Antigen präsentierenden Zellen und IL-2 expandiert werden. Die expandierten T-Zellen werden nach Transfer in vivo spezifisch die virusinfizierten Zellen oder die Tumorzellen, die das Antigen exprimieren, attackieren.

Mit Hilfe des EBV lassen sich von jedem Individuum auf relativ einfache Weise Zellinien etablieren. Diese Zellen können ex vivo mit Genkonstrukten versehen und anschließend wieder in das syngene Individuum transplantiert werden. Die Zellen können daher bei einer adoptiven Immuntherapie entweder als Zytokinproduzenten oder als Antigen präsentierende Zellen zum Einsatz kommen. EBV ist in allen Ausführungsformen auch durch mini-EBV ersetzbar.

Zusammenfassend wird somit ein bevorzugt auf EBV basierender Vektor bereitgestellt, der besser als alle anderen bekannten Vektorsysteme ermöglicht, in Zellen, insbesondere in B-Zellen, eine hohe und dauerhafte, d.h. stabile Expression zu erreichen. Therapeutisch wertvolle Gene wie Zytokin-Gene oder Tumorantigene können stabil in die zu behandelnden B-Zellen aufgenommen und zur Therapie maligner und viraler Erkrankungen des Menschen, insbesondere von Erkrankungen, die mit Tumoren der B-Zellen einhergehen, eingesetzt werden. Die erfindungsgemäßen Genkonstrukte eignen sich insbesondere zur gentherapeutischen Anwendung.

Die Erfindung umfaßt somit auch eine pharmazeutische Zubereitung, die autologe T-Zellen enthält, die ex vivo unter Verwendung primärer, mit EBV oder mini-EBV- oder deren Derivaten immortalisierter B-Lymphozyten oder Fibroblasten, die mit einem erfindungsgemäßen Genskonstrukt modifiziert, stimuliert und expandiert wurden.

Die Erfindung umfaßt auch ein Verfahren zur in vitro Modifikation primärer, mit EBV oder mini-EBV- oder deren Derivaten immortalisierter B-Lymphozyten oder Fibroblasten, wobei ein oder mehrere der erfindungsgemäßen Genkonstrukte zum Gentransfer in die B-Lymphozyten oder Fibroblasten verwendet werden.

Erfindungsgemäß wird auch ein Verfahren zur Wachstumsstimulation autologer T-Zellen ex vivo offenbart, wobei in primäre, mit EBV oder mini-EBV- oder deren Derivaten immortalisierte B-Lymphozyten oder Fibroblasten eine oder mehrere der erfindungsgemäßen Genkonstrukte durch Gentransfer in die B-Lymphozyten oder Fibroblasten eingebracht und mit autologen T-Zellen ex vivo zur Wachstums-Stimulation in Kontakt gebracht werden.

Eukaryontenzellen, die die erfindungsgemäßen Genkonstrukte in integrierter oder nicht-integrierter Form enthalten, können beispielsweise zur Produktion von Zytokinen oder als Tumorantigen präsentierende Zellen verwendet werden.

### Literatur

Belt, P.B., Groeneveld, H., Teubel, W.J., van-de-Putte, P., and Backendorf, C. (1989). Construction and properties of an Epstein-Barr-virus-derived cDNA expression vector for human cells. Gene *84,* 407-417.

Belt, P.B., Jongmans, W., de-Wit, J., Hoeijmakers, J.H., van-de-Putte, P., and Backendorf, C. (1991). Efficient cDNA cloning by direct phenotypic correction of a mutant human cell line (HPRT-) using an Epstein-Barr virus-derived cDNA expression vector. Nucleic. Acids. Res. *19,* 4861-4866.

Boon, T., Cerottini, J.-C., Van den Eynde, B., van der Bruggen, P., and Van Pel, A. (1994). Tumor antigens recognized by T lymphoytes. Annu. Rev. Immunol. *12,* 337-365.

Cleary, M.L., Epstein, M.A., Finerty, S., Dorfman, R.F., Bornkamm, G.W., Kirkwood, J.K., Morgan, AJ., and Sklar, J. (1985). Individual tumors of multifocal EB virus-induced malignant lymphomas in tamarins arise from different B-cell clones. Science *228,* 722-724.

Cox, A.L., Skipper, J., Chen. Y., Henderson, R.A., Darrow, T.L., Shabanowitz, J., Engelhard, V.H., Hunt, D.F., and Slingluff, C.L., Jr. (1994). Identification of a peptide recognized by five melanoma-specific human cytotoxic T cell lines. Science *264,* 716-719.

Culver, K.W. and Blaese, R.M. (1994). Gene therapy for cancer. Trends Genet. *10,* 174-178.

Culver, K.W. and Van Gilder, J. (1994). Gene therapy for the treatment of malignant brain tumors with *in vivo* tumor transduction with the herpes simplex thymidine kinase gene/ganciclovir system. Hum. Gene Ther. *5,* 343-379.

Dranoff, G., Jaffee, E., Lazenby, A., Golumbek, P., Levitsky, H., Brose, K., Jackson, V., Hamada, H., Pardoll, D., and Mulligan, R.C. (1993). Vaccination with irradiated tumor cells engineered to secrete murine granulocyle-macrophage colony-stimulating factor stimulates potent, specific, and long-lasting anti-tumor immunity. Proc. Natl. Acad. Sci. U. S. A. *90,* 3539-3543.

Feichtinger, H., Li, S.L., Kaaya, E., Putkonen, P., Grunewald, K., Weyrer, K., Bottiger, D., Ernberg, I., Linde, A., Biberfeld, G., and et-al (1992). A monkey model for Epstein Barr virus-associated lymphomagenesis in human acquired immunodeficiency syndrome [published erratum appears in J Exp Med 1992 Aug 1;176(2):following 634]. J. Exp. Med. *176,* 281-286.

Foa, R., Guarini, A., Cignetti, A., Cronin, K., Rosenthal, F., and Gansbacher, B. (1994). Cytokine gene therapy: A new strategy for the management of cancer patients. Nat. Immun. *13,* 65-75.

Fulton, R. and Van Ness, B. (1993). Kappa immunoglobulin promoters and enhancers display developmentally controlled interactions. Nucleic Acids Res. *21,* 4941-4947.

Hotchin, N.A., Allday, M.J., and Crawford, D.H. (1990). Deregulated c-myc expression in Epstein-Barr-virus-immortalized B-cells induces altered growth properties and surface phenotype but not tumorigenicity. Int. J. Cancer *45,* 566-571.

Hotchin, N.A., Wedderbum, N., Roberts, I., Thomas, J.A., Bungey, J.A., Naylor, B., and Crawford, D.H. (1993). Analysis of the tumorigenic potential of common marmoset lymphoblastoid cells expressing a constitutively activated c-myc gene. Br. J. Cancer *67,* 926-932.

June, C.H., Bluestone, J.A., Nadler, L.M., and Thompson, C.B. (1994). The B7 and CD28 receptor families. Immunol. Today *15,* 321-331.

Li, S.L., Feichtinger, H., Haaya, E., Migliorini, P., Putkonen, P., Biberfeld, G., Middeldorp, J.M., Biberfeld, P., and Ernberg, I. (1993). Expression of Epstein-Barr-virus-related nuclear antigens and B-cell markers in lymphomas of SIV-immunosuppressed monkeys. Int. J. Cancer *55,* 609-615.

Li, Y., McGowan, P., Hellström, I., Hellström, K.E., and Chen, L. (1994). Costimulation of tumor-reactive CD4⁺ and CD8⁺ T lymphocytes by B7, a natural ligand for CD28, can be used to treat established mouse melanoma. J. Immunol. *153,* 421-428.

Mandelboim, O., Berke, G., Fridkin, M., Feldman, M., Eisenstein, M., and Eisenbach, L. (1994). CTL induction by a tumour-associated antigen octapeptide derived from a murine lung carcinoma. Nature *369,* 67-71.

Margolskee, R.F. (1992). Epstein-Barr virus based expression vectors. Curr. Top. Microbiol. Immunol. *158,* 67-95.

Margolskee, R.F., Kavathas, P., and Berg, P. (1988). Epstein-Barr virus shuttle vector for stable episomal replication of cDNA expression libraries in human cells. Mol. Cell Bio. *8,* 2837-2847.

Middleton, T. and Sugden, B. (1994). Retention of plasmid DNA in mammalian cells is enhanced by binding of the Epstein-Barr virus replication protein EBNA1. J. Virol. *68,* 4067-4071.

Nabel, G.J., Nabel, E.G., Yang, Z., Fox, B.A., Plautz, G.E., Gao, X., Huang, L., Shu, S., Gordon, D., and Chang, A.E. (1993). Direct gene transfer with DNA-liposome complexes in melanoma: Expression, biologic activity, and lack of toxicity in humans. Proc. Natl. Acad. Sci. USA *90,* 11307-11311.

Ohno, T., Gordon, D., San, H., Pompili, V.J., Imperiale, M.J., Nabel, G.J., and Nabel, E.G. (1994). Gene therapy for vascular smooth muscle cell proliferation after arterial injury. Science *265,* 781-784.

Pardoll, D.M. (1993). Cancer vaccines. Immunol. Today *14,* 310-316.

Plautz, G.E., Yang. Z.Y., Wu, B.Y., Gao, X., Huang, L., and Nabel, G.J. (1993). Immunotherapy of malignancy by in vivo gene transfer into tumors [see comments]. Proc. Natl. Acad. Sci. U. S. A. *90,* 4645-4649.

Rogers, R.P., Strominger, J.L., and Speck, S.H. (1992). Epstein-Barr virus in B lymphocytes: viral gene expression and function in latency. Adv. Cancer Res. *58,* 1-26.

Treisman, J., Hwu, P., Minamoto, S., Shafer, G.E., Cowherd, R., Morgan, R.A.. and Rosenberg, S.A. (1995). Interleukin-2-transduced lymphocytes grow in an autocrine fashion and remain responsive to antigen. Blood *85,* 139-145.

Yates, J., Warren, N., Reisman, D., and Sugden, B. (1984). A cis-acting element from the Epstein-Barr viral genome that permits stable replication of recombinant plasmids in latently infected cells. Proc. Natl. Acad. Sci. U. S. A. *81,* 3806-3810.

Yates, J.L., Warren. N., and Sugden, B. (1985). Stable replication of plasmids derived from Epstein-Barr virus in various mammalian cells. Nature *313,* 812-815.

Hörtnagel, K., Mautner, J., Strobl, L., Wolf, D., Christoph, B., Geltinger, C., and Polack, A. (1995). The role of immunoglobulin kappa elements in c-myc activation. Oncogene *10,* 1393-1401.

Polack, A., Feederle, R., Klobeck, G., and Hörtnagel, K. (1993). Regalatory elements in the immunoglobulin kappa locus induce c-myc activation and the promoter shift in Burkitt's lymphoma cells. EMBO J. *12,* 3913-3920.

Sugden B, Marsh K and Yates J. (1985). Mol. Cell Biol., 5, 410 - 413.

## Patentansprüche

1. Genkonstrukt, mindestens enthaltend, in funktioneller Verbindung,
(a)
(i) eine Kombination von zwei Enhancerelementen des Immunglobulin-κ-Locus, nämlich den κ-Intron-Enhancer (κ-Ei) und den κ-3'-Enhancer (κ-E3'), oder
(ii) eine Kombination von zwei Enhancerelementen des schwere Ketten-Immunglobulin-µ-Locus, nämlich µ-Ei und die 3' von Cα gelegene µ-E3'-Enhancerregion, oder
(iii) eine Kombination eines oder mehrerer dieser Enhancerelemente aus (ii) mit einem oder mehreren der vorgenannten Elemente des Immunglobulin-κ-Locus oder
(iv) das eine Enhancerelement aus dem Immunglobulin-λ-Locus oder
(v) eine Kombination dieses Enhancerelements aus (iv) mit einem oder mehreren der vorgenannten Elemente des Immunglobulin-κ-Locus oder
(vi) eine Kombination dieses Enhancerelements aus
(iv) mit einem oder mehreren der vorhergehenden Elemente des schwere Ketten-Immunglobulin-µ-Locus, weiterhin
(b) einen Promotor,
(c) ein Gen von Interesse, ausgewählt aus mindestens einem Element der Gruppe, bestehend aus Zytokin-Gen, virales Antigen, Zelladhäsions-Gen, Tumorantigen, co-stimulatorisches Signalmolekül-Gen, ein nicht mit dem Empfänger identisches HLA-Gen, und β-Galactosidase-Gen, ausgenommen das c-myc-Gen, und
(d) eine Polyadenylierungsstelle (PAA).

2. Genkonstrukt nach Anspruch 1,
**dadurch gekennzeichnet,**
daß die Kombination der Regulationselemente aus den nachfolgenden Kombinationen ausgewählt wird:
κ-Ei mit λ-E, µ-Ei mit κ-E3', µ-Ei mit µ-E3', κ-Ei mit µ-E3', µ-Ei mit λ-E, κ-MAR mit κ-Ei mit κ-E3', λ-E mit κ-E3', λ-E mit µ-E3'.

3. Genkonstrukt nach Anspruch 1 oder 2,
**dadurch gekennzeichnet,**
daß es zusätzlich zu einem oder mehreren der Enhancerelemente oder der Kombinationen der Enhancerelemente nach Anspruch 1 oder 2 die Immunglobulin-κ-Matrix-Attachment-Region (κ-MAR) oder die Immunglobulin-µ-Matrix-Attachment-Region (µ-MAR) enthält.

4. Genkonstrukt nach einem oder mehreren der vorhergehenden Ansprüche,
**dadurch gekennzeichnet,**
daß es weiterhin Sequenzen enthält, die aus Sequenzen von EBV-Vektoren, mini-EBV-Vektoren, bakteriellen Vektoren, Retroviren, mit Adenoviren assoziierten Viren, Adenoviren und Vaccinia-Viren abgeleitet sind.

5. Genkonstrukt nach einem oder mehreren der vorhergehenden Ansprüche,
**dadurch gekennzeichnet,**
daß die von EBV-Vektoren abgeleitete Sequenz der Replikationsursprung (oriP) oder EBNA1 ist.

6. Genkonstrukt nach einem oder mehreren der vorhergehenden Ansprüche,
**dadurch gekennzeichnet,**
daß es weiterhin ein Markergen enthält.

7. Genkonstrukt nach einem oder mehreren der vorhergehenden Ansprüche,
**dadurch gekennzeichnet,**
daß das Markergen ein Resistenzgen, bevorzugt ein Ampicillin- oder Hygromycinresistenzgen oder Neomycinresistenzgen und/oder die bakterielle Vektorsequenz von pBR-Vektoren abgeleitet ist und/oder die bakterielle Vektorsequenz und/oder das Markergen in EBV-Vektoren enthalten sind.

8. Genkonstrukt nach einem oder mehreren der vorhergehenden Ansprüche,
**dadurch gekennzeichnet,**
daß die von EBV-Vektoren abgeleitete Sequenz den EBV-Replikationsursprung (oriP), ein Resistenzgen und von pBR-Vektoren abgeleitete Sequenzen, wahlweise zusätzlich die Expressionskassette für EBNA1, umfaßt.

9. Genkonstrukt nach einem oder mehreren der vorhergehenden Ansprüche,
**dadurch gekennzeichnet,**
daß der Promotor aus der Gruppe B-Zell-spezifischer Promotoren, Zytomegalievirus-(CMV-)-, tk- und β-Globin-Promotoren und c-myc-P1-Promotor ausgewählt ist.

10. Prokaryonten- oder Eukaryontenzelle,
**dadurch gekennzeichnet,**
daß sie ein oder mehrere der Genkonstrukte nach einem oder mehreren der vorhergehenden Ansprüche in integrierter oder nicht-integrierter (episomaler) Form enthält.

11. Pharmazeutische Zubereitung,
**dadurch gekennzeichnet,**
daß sie ein oder mehrere der Genkonstrukte nach einem oder mehreren der vorhergehenden Ansprüche in einer wirksamen Menge neben üblichen Träger- und/oder Hilfsstoffen enthält.

12. Verwendung eines Genkonstrukts nach einem oder mehreren der vorhergehenden Ansprüche zur Herstellung einer pharmazeutischen Zubereitung, die mit einem solchen Genkonstrukt modifizierte primäre, mit EBV oder mini-EBV- oder deren Derivaten immortalisierte B-Lymphozyten oder Fibroblastenzellen in einer wirksamen Menge neben üblichen Träger- und/oder Hilfsstoffen enthält.

13. Verwendung eines Genkonstrukts nach einem oder mehreren der vorhergehenden Ansprüche zur Herstellung einer pharmazeutischen Zubereitung, die mit einem solchen Genkonstrukt modifizierte primäre, mit EBV oder mini-EBV- oder deren Derivaten immortalisierte B-Lymphozyten oder Fibroblastenzellen zusammen mit autologen T-Zellen in einer wirksamen Menge neben üblichen Träger- und/oder Hilfsstoffen enthält.

14. Verwendung eines Genkonstrukts nach einem oder mehreren der vorhergehenden Ansprüche zur Herstellung einer pharmazeutischen Zusammensetzung für die Gentherapie zur Behandlung von Tumoren, zur direkten Inokulation in Tumore, zur Stimulation einer Immunreaktion oder zur Modifikation primärer, EBV-immortalisierter B-Lymphozyten.

15. Verwendung von primären, mit EBV oder mini-EBV- oder deren Derivaten immortalisierten B-Lymphozyten oder Fibroblasten, die mit einem Genkonstrukt nach einem oder mehreren der vorhergehenden Ansprüche modifiziert wurden, zur Wachstumsstimulation autologer T-Zellen ex vivo.

16. Verwendung von ein Genkonstrukt nach einem oder mehreren der vorhergehenden Ansprüche in integrierter oder nichtintegrierter Form enthaltenden Eukaryontenzellen zur Herstelllung eines Medikaments zur Immuntherapie.
